# FASCICULE DE BREVET EUROPEEN

(11) **EP 0 648 485 B1**
(45) Date de publication et mention de la délivrance du brevet: **23.12.1998**
(21) Numéro de dépôt: 94402302.7
(22) Date de dépôt: 14.10.1994
(51) Int. Cl.: A61K 7/043

(54) **Vernis à ongles aqueux, contenant des particules de polyester-polyuréthanne anionique à l'état dispersé**
Wässriger Nagellack enthaltend dispergierte Partikel eines anionischen Polyester-polyurethans
Aqueous nail varnish containing dispersed particles of anionic polyester-polyurethane

(30) Priorité: 15.10.1993 FR 9312272
(43) Date de publication de la demande: 19.04.1995
(73) Titulaire: L'OREAL, 75008 Paris (FR)
(72) Inventeur: de La Poterie, Valérie, F-77820 Le Chatelet en Brie (FR)
(74) Mandataire: Stalla-Bourdillon, Bernard

(56) Documents cités:
- EP-A- 0 143 480
- EP-A- 0 391 322
- EP-A- 0 423 471

## Description

La présente invention a pour objet une composition cosmétique sous forme de vernis à ongles aqueux, coloré ou incolore, contenant en tant que substance filmogène des particules de polyester-polyuréthanne anionique à l'état dispersé, et dont la taille des particules est comprise entre 2 et 40 nm.

A l'heure actuelle, la majeure partie des compositions se présentant sous forme de vernis à ongles sont à base d'un mélange de solvants organiques contenant de la nitrocellulose, une résine aryle-sulfonamide formaldéhyde ou une résine alkyde et un agent plastifiant. De tels vernis, du fait de la présence de solvants organiques, présentent un certain nombre d'inconvénients dans la mesure où ils peuvent endommager les ongles ou la cuticule et provoquer par ailleurs, lors de l'application ou du séchage certains dangers pour leurs utilisatrices. Ils peuvent également présenter des risques inflammabilité.

Ainsi, depuis quelques années, les recherches se sont donc orientées vers la mise au point de vernis à ongles exempts de solvants organiques et en particulier de vernis aqueux.

Pour ce faire, différentes substances filmogènes ont été envisagées.

Il a été décrit dans la demande de brevet EP n° 143.480, l'utilisation, dans des compositions de vernis à ongles aqueux, de dispersions de polyuréthanne dont le seul exemple figurant dans cette demande, est une dispersion commercialisée sous la dénomination de "Néorez R 974" par la Société ICI et dont la taille des particules est de 68 nm.

Il a également été décrit dans la demande de brevet EP 423.471, l'utilisation de dispersions de polyester-polyuréthanne dont la taille des particules est inférieure à 200 nm.

Il s'est avéré toutefois que les vernis à ongles aqueux obtenus à partir de telles dispersions étaient insuffisamment rémanents à l'eau, c'est à dire qu'ils avaient tendance à s'éliminer par simple lavage des mains à l'eau.

On a maintenant constaté après de nombreuses études, qu'il était possible d'améliorer dans des conditions particulièrement significatives la rémanence à l'eau des vernis à ongles aqueux en utilisant à titre de substances filmogènes des dispersions de particules de polyester-polyuréthannes, dont la taille des particules est comprise entre 2 et 40 nm, tout en conservant une brillance et une aptitude à l'étalement du vernis sur la surface des ongles tout à fait satisfaisantes.

La présente invention a donc pour objet à titre de produit industriel nouveau un vernis à ongles aqueux, coloré ou incolore, caractérisé par le fait qu'il contient des particules de polyester-polyuréthanne anionique à l'état dispersé, dont la taille de particules est comprise entre 2 et 40 nm et la dureté, mesurée au pendule de Persoz, du film obtenu après séchage, durant 24 heures à 30°C et à 50 % d'humidité relative, d'une couche de 300 µm d'épaisseur d'une dispersion aqueuse à 28 % de matière sèche desdites particules est comprise entre 50 et 300 secondes.

Selon l'invention, la taille des particules a été déterminée à l'aide d'un appareil commercialisé sous la dénomination de "BI-90" par la Société Brookhaven Instruments Corporation.

De préférence, la taille des particules utilisées dans les vernis à ongles selon l'invention est comprise entre 2 et 30 nm.

La dureté a été déterminée selon la méthode dite du pendule de Persoz et est décrite dans la norme NF-T-30-016.

De préférence, la dureté définie précédemment est comprise entre 80 et 250 secondes.

Selon un mode de réalisation préféré des vernis à ongles selon l'invention, la proportion en particules de polyester-polyuréthanne à l'état dispersé est comprise entre 3 et 50 % et de préférence entre 10 et 50 % par rapport au poids total du vernis.

Les particules de polyester-polyuréthanne utilisées selon l'invention sont généralement commercialisées sous forme de dispersions aqueuses.

La teneur en particules de polyester-polyuréthanne desdites dispersions actuellement disponibles sur le marché, est comprise entre environ 30 et 50 % en poids par rapport au poids total de la dispersion.

Parmi les dispersions de polyester-polyuréthanne anionique utilisables dans les vernis à ongles aqueux selon l'invention, on peut citer en particulier celles commercialisées sous les dénominations de "Sancure 2060" et "Sancure 815" par la Société Sanncor.

Selon un mode de réalisation particulier des vernis à ongles aqueux selon l'invention, lesdits vernis contiennent en outre des particules de polyéther-polyuréthanne anionique à l'état dispersé, dont la taille desdites particules est comprise entre 30 et 500 nm et de préférence entre 50 et 150 nm.

La proportion en particules de polyéther-polyuréthanne à l'état dispersé est généralement inférieure à celle des particules de polyester-polyuréthanne.

Les particules de polyéther-polyuréthanne sont également commercialisées sous forme de dispersions aqueuses.

Parmi les dispersions de particules de polyéther-polyuréthanne, on utilise de préférence, celles pour lesquelles le film obtenu après séchage possède une énergie de surface, mesurée par la méthode de l'angle de contact, comprise entre 15 et 45 mj/m².

La méthode de l'angle de contact consiste à déposer une goutte d'eau et une goutte de diiodométhane sur ledit film, à mesurer l'angle formé par chaque goutte avec le film, puis à calculer l'énergie de surface dudit film d'après la méthode décrite dans "Double liaison - Chim. Peint. 82." Vol. 29, pages 263 à 268.

Parmi les dispersions de particules de polyéther-polyuréthanne anionique utilisables selon l'invention, on peut citer en particulier celles commercialisées sous la dénomination de "Sancure 878" par la Société Sanncor, et sous la dénomination de "Néorez R 970" par la Société ICI.

Le caractère anionique des polyester-polyuréthannes et des polyéther-polyuréthannes utilisés selon l'invention est du à la présence dans leurs motifs constitutifs de groupements à fonction acide carboxylique ou acide sulfonique.

Selon un mode de réalisation particulier des vernis à ongles selon l'invention, on peut utiliser un mélange de dispersions commerciales constitué de particules de polyester-polyuréthanne anionique telles que définies ci-dessus et de particules de polyéther-polyuréthanne anionique également définies ci-dessus.

Par exemple, on peut utiliser un mélange constitué de la dispersion commercialisée sous la dénomination de "Sancure 2060" et de celle commercialisée sous la dénomination de "Sancure 861" ou un mélange de celle commercialisée sous la dénomination de "Sancure 815" et de celle commercialisée sous la dénomination de "Sancure 878", ces dispersions étant commercialisées par la Société Sancor. On utilise de préférence selon l'invention, des mélanges contenant respectivement 60 % et 70 % de particules de polyester-polyuréthanne, le reste étant constitué par des particules de polyéther-polyuréthanne.

Les vernis à ongles selon l'invention peuvent contenir en outre au moins un agent épaississant en une proportion comprise entre 0,01 et 5 % et de préférence entre 0,1 et 1 % en poids par rapport au poids total du vernis.

Parmi les agents épaississants appropriés pour la formulation des vernis à ongles aqueux, on peut citer la cellulose et ses dérivés telles que la carboxyméthylcellulose et l'hydroxyéthylcellulose, les silicates, les argiles telles que la laponite, les polymères synthétiques tels que les polymères acryliques ou les polymères associatifs de type polyuréthanne et les gommes naturelles telles que la gomme de carraghénane ou de xanthane. On utilise de préférence un agent épaississant choisi parmi l'hydroxyéthylcellulose, la laponite, et les polyuréthannes associatifs.

Lorsque les vernis à ongles selon l'invention sont colorés, ils contiennent alors au moins un pigment organique ou inorganique en une proportion comprise entre 0,01 et 5 % en poids et de préférence entre 0,5 et 2 % en poids par rapport au poids total du vernis.

Parmi les pigments organiques, on peut citer les D et C Red n° 10, 11, 12 et 13, le D et C Red n° 7, les D et C Red n° 5 et 6, les D et C Red n° 30 et 34, des laques telles que la laque D et C yellow n° 5 et la laque D et C Red n° 2, ou encore la guanine.

Parmi les pigments inorganiques, on peut citer : le dioxyde de titane, l'oxychlorure de bismuth, l'oxyde de fer brun et les oxydes de fer rouge.

On peut en outre modifier l'aptitude à l'étalement sur les ongles du vernis en utilisant des tensioactifs fluorés hydrosolubles. Parmi ceux-ci, on peut citer notamment ceux correspondant aux formules (I), (II) et (III) suivantes :
1)

   (CₙF₂ₙ₊₁)-C₂H₄X (I)

   dans laquelle :
   le radical CₙF₂ₙ₊₁ est linéaire ou ramifié,
   n est compris entre 3 et 16,
   X représente un radical choisi parmi :
      (i) -CO₂Y,
      (ii) -SO₃Y
         Y représentant un atome d'hydrogène, un métal alcalin ou un groupe aminé tel qu'un groupe ammonium,
      (iii) -(OC₂H₄)ₘ-OH
         m étant compris entre 2 et 100, de préférence entre 4 et 40,
      (iv) 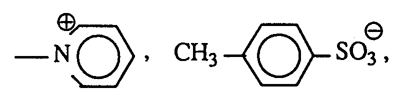
      (v) -SO₂NH(CH₂)₃-N^{⊕}(CH₃)₃I^{⊖},
      (vi) 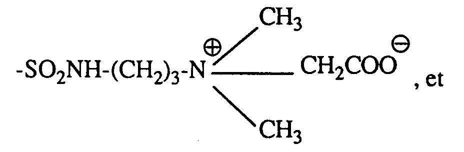
      (vii) 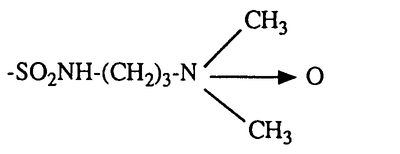
      (viii) -SCH₂CH₂R
         R représentant soit le radical CO₂M, M étant un métal alcalin, en particulier le lithium, soit le radical N^{⊕}(CH₃)₃CH₃SO₄^{⊖}.
2)

   (CₙF₂ₙ₊₁)-R₁ (II)

   dans laquelle :
   le radical CₙF₂ₙ₊₁ est linéaire ou ramifié,
   n étant compris entre 4 et 16,
   R₁ est un radical choisi parmi :
      SO₃^{⊖} NH₄^{⊕}, CO₂^{⊖} NH₄^{⊕}, SO₃^{⊖} N^{⊕}(R₃)₄, et CO₂^{⊖} N^{⊕} (R₃)₄, R₃ étant un radical alkyle en C₁-C₄ ou
   R₁ représente un radical répondant à l'une des formules suivantes :
      (i) -SO₂N(R₃)CH₂-CO₂^{⊖}X^{⊕}
      R₃ tel que défini ci-dessus, et
      X est un atome d'hydrogène ou un métal alcalin,
      (ii) -SO₂-NH(CH₂)ₚN^{⊕}(R₃)₃I^{⊖}
      p étant un nombre entier compris entre 1 et 4, et
      R₃ étant et tel que défini ci-dessus, et
      (iii) -SO₂-N(R₃)(CH₂CH₂OY)
      Y étant un atome d'hydrogène ou un radical alkyle en C₁-C₄, et
      R₃ étant tel que défini ci-dessus.
3)

   (CₙF₂ₙ₊₁C₂H₄O)ₓP(O)(R)_{y} (III)

   n étant compris entre 3 et 8,
   x et y, différents, étant 1 ou 2, et
   R représente ONH₄ ou OH.

Parmi les composés perfluoroalkyles de formule (I), on peut notamment citer ceux correspondant aux formules suivantes :
a) 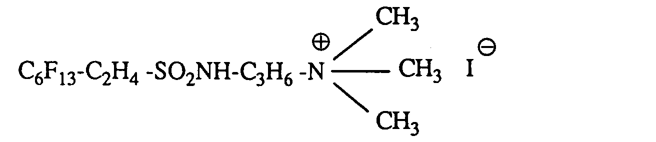
   commercialisé sous la dénomination de "Forafac 1179" par la Société Atochem ;
b) 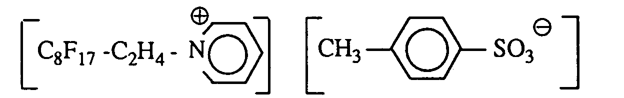
   commercialisé sous la dénomination de "Forafac 1098" par la Société Atochem ;
c) 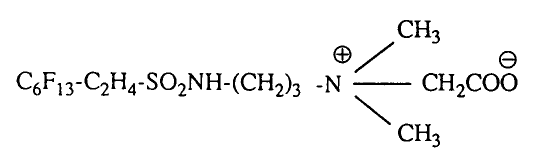
   commercialisé sous la dénomination de "Forafac 1157" par la Société Atochem ;
d) CₙF₂ₙ₊₁CH₂CH₂O(CH₂CH₂O)ₓH
   n = 3 à 8 et
   x = 2 à 100
   commercialisé sous les dénominations de "Zonyl-FSN" et "Zonyl.FSN100" par la Société Du Pont;
e) CₙF₂ₙ₊₁CH₂CH₂SCH₂CH₂CO₂Li
   n= 3 à 8
   commercialisé sous la dénomination de "Zonyl FSA" par la Société Du Pont ; et
f) CₙF₂ₙ₊₁CH₂CH₂SCH₂CH₂N^{⊕}(CH₃)₃CH₃SO₄^{⊖}
   n = 3 à 8
   commercialisé sous la dénomination de "Zonyl FSC" par la Société Du Pont.

Parmi les composés perfluoroalkyles de formule (II), on peut notamment citer :
a) CₙF₂ₙ₊₁SO₂N(C₂H₅)CH₂CO₂^{⊖}K^{⊕}
   n = 8
   commercialisé sous la dénomination de "Fluorad FC 129" par la Société 3M ;
b) CₙF₂ₙ₊₁SO₂NHC₃H₆N^{⊕}(CH₃)₃I^{⊖}
   n = 8
   commercialisé sous la dénomination de "Fluorad FC 135" par la Société 3M ;
c) CₙF₂ₙ₊₁SO₂N(C₂H₅)(CH₂CH₂OH)
   n = 8
   commercialisé sous la dénomination de "Fluorad FC 170C" par la Société 3M ;
d) CₙF₂ₙ₊₁SO₃ NH₄^{⊕}
   n = 10
   commercialisé sous la dénomination de "Fluorad FC 120" par la Société 3M ;
e) CₙF₂ₙ₊₁SO₂N(C₂H₅)CH₂CO₂^{⊖}NH₄^{⊕}
   n = 8
   commercialisé sous la dénomination de "Fluorad FC 143" par la Société 3M.

Parmi les composés perfluoroalkyles de formule (III), on peut notamment citer :
a) (CₙF₂ₙ₊₁CH₂CH₂O)₁₋₂P(O)(ONH₄)₂₋₁
   n = 3 à 8
   commercialisé sous la dénomination de "Zonyl FSP, PSE" par la Société Du Pont; et
b) (CₙF₂ₙ₊₁CH₂CH₂O)₁₋₂P(O)(OH)₂₋₁
   n = 3 à 8
   commercialisé sous la dénomination de "Zonyl UR" par la Société Du Pont.

La proportion de tensio-actifs fluorés hydrosolubles peut être comprise entre 0,01 et 1% en poids par rapport au poids total du vernis à ongles, et de préférence entre 0,05 et 0,2%.

Les vernis à ongles, selon l'invention, peuvent contenir en outre au moins un additif choisi parmi un agent mouillant, un agent dispersant, un anti-mousse, un filtre solaire, un conservateur, un accélérateur de séchage, une cire, une silicone ou un mélange de ceux-ci.

On va maintenant donner à titre d'illustration plusieurs exemples de vernis à ongles aqueux selon l'invention.

### EXEMPLE 1:Vernis à ongles coloré

| | |
|---|---|
| - Dispersion aqueuse de polyester-polyuréthanne anionique à 30% commercialisée sous la dénomination de "Sancure 2060" par la Société Sanncor | 93,3 % |
| - Epaississant associatif polyuréthanne commercialisé sous la dénomination de "Ser AD FX 1100" par la Société Servo | 0,30 % |
| - Pigments | 1 % |
| - Conservateurs | 0,05 % |
| - Eau qsp | 100 % |

Le vernis obtenu s'étale facilement sur l'ongle et présente après séchage une dureté très satisfaisante.

La résistance à l'eau du vernis obtenu a été évaluée en appliquant un film de 300 µm sur une plaque de verre puis en la plongeant pendant une heure avec agitation, dans de l'eau froide ou chaude (45°C) avec ou sans détergent. On n'a observé alors aucune décoloration, pas plus qu'un déchirement, ou même une dissolution du film dans le temps.

Le vernis obtenu présente donc une excellente résistance à l'eau, notamment chaude même en présence d'un détergent.

### EXEMPLE 2: Vernis à ongles coloré

| | |
|---|---|
| - Dispersion aqueuse de polyester-polyuréthanne anionique à 35 % commercialisée sous la dénomination de "Sancure 815" par la Société Sanncor | 85,7 % |
| - Epaississant associatif polyuréthanne commercialisé sous la dénomination de "Ser AD FX 1100" par la Société Servo | 0,30 % |
| - Pigments | 1 % |
| - Conservateurs | 0,05 % |
| - Eau qsp | 100 % |

### EXEMPLE 3: Vernis à ongles coloré

| | |
|---|---|
| - Dispersion aqueuse de polyester-polyuréthanne anionique à 30 % commercialisée sous la dénomination de "Sancure 2060" par la Société Sanncor | 65,3 % |
| - Dispersion aqueuse de polyéther-polyuréthanne anionique à 40 % commercialisée sous la dénomination de "Sancure 861" par la société Sanncor | 21,0 % |
| - Epaississant associatif polyuréthanne commercialisé sous la dénomination de "Ser AD FX 1100" par la Société Servo | 0,30 % |
| - Pigments | 1 % |
| - Conservateurs | 0,05 % |
| - Eau qsp | 100 % |

### EXEMPLE 4: Vernis à ongles coloré

| | |
|---|---|
| - Dispersion aqueuse de polyester-polyuréthanne anionique à 35 % commercialisée sous la dénomination de "Sancure 815" par la Société Sanncor | 56,0 % |
| - Dispersion aqueuse de polyéther-polyuréthanne anionique à 38 % commercialisée sous la dénomination de "Sancure 878" par la Société Sanncor | 22,1 % |
| - Epaississant associatif polyuréthanne commercialisé sous la dénomination de "Ser AD FX 1100" par la Société Servo | 0,30 % |
| - Pigments | 1 % |
| - Conservateurs | 0,05 % |
| - Eau qsp | 100 % |

Le vernis obtenu présente, après séchage, une dureté et un résistance à l'eau très satisfaisante.

### EXEMPLE 5: Vernis à ongles coloré

| | |
|---|---|
| - Dispersion aqueuse de polyester-polyuréthanne anionique à 30 % commercialisée sous la dénomination de "Sancure 2060" par la Société Sanncor | 93,3 % |
| - Epaississant associatif polyuréthanne non ionique commercialisé sous la dénomination de "Dapral T 210" par la Société Akzo | 1,50 % |
| - Silicate de sodium et de magnésium commercialisé sous la dénomination de "Laponite XLG" par la Société Laporte | 0,20 % |
| - Tensioactif fluoré commercialisé sous la dénomination de "Fluorad FC-143" par la Société 3M | 0,10 % |
| - Pigments | 1,50 % |
| - Eau qsp | 100 % |

Le vernis obtenu s'étale facilement sur l'ongle et présente après séchage une dureté, et une résistance à l'eau très satisfaisante.

## Revendications

1. Vernis à ongles aqueux, coloré ou incolore, caractérisé par le fait qu'il contient des particules de polyester-polyuréthanne anionique à l'état dispersé, dont la taille des particules est comprise entre 2 et 40 nm et la dureté, mesurée au pendule de Persoz, du film obtenu après séchage, durant 24 heures à 30°C et à 50 % d'humidité relative, d'une couche de 300 µm d'épaisseur d'une dispersion aqueuse à 28 % de matière sèche desdites particules est comprise entre 50 et 300 secondes.

2. Vernis à ongles aqueux selon la revendication 1, caractérisé par le fait que la taille desdites particules est comprise entre 2 et 30 nm.

3. Vernis à ongles aqueux selon l'une quelconque des revendications précédentes caractérisé par le fait que la dureté telle que définie à la revendication 1 est comprise entre 80 et 250 secondes.

4. Vernis à ongles selon l'une quelconque des revendications précédentes, caractérisé par le fait que la proportion de particules de polyester-polyuréthanne à l'état dispersé dans ledit vernis à ongles est comprise entre 3 et 50 % et de préférence entre 10 et 50 % en poids par rapport au poids total du vernis.

5. Vernis à ongles selon l'une quelconque des revendications précédentes, caractérisé par le fait qu'il contient en outre des particules de polyéther-polyuréthanne anionique à l'état dispersé, la taille desdites particules étant comprise entre 30 et 500 nm et de préférence entre 50 et 150 nm.

6. Vernis à ongles selon la revendication 5, caractérisé par le fait que la proportion en poids desdites particules de polyéther-polyuréthanne est inférieure à celle des particules de polyester-polyuréthanne.

7. Vernis à ongles selon l'une quelconque des revendications précédentes, caractérisé par le fait qu'il contient en outre au moins un agent épaississant en une proportion comprise entre 0,01 et 5 % et de préférence entre 0,01 et 1 % en poids par rapport au poids total du vernis.

8. Vernis à ongles selon la revendication 7, caractérisé par le fait que ledit agent épaississant est choisi dans le groupe constitué par la cellulose et ses dérivés, les silicates, les argiles, les polymères synthétiques et les gommes naturelles.

9. Vernis à ongles selon la revendication 8, caractérisé par le fait que ledit agent épaississant est choisi dans le groupe constitué par l'hydroxyéthylcellulose, la laponite et les polyuréthannes associatifs.

10. Vernis à ongles selon l'une quelconque des revendications précédentes, caractérisé par le fait qu'il contient en outre au moins un pigment organique ou inorganique en une proportion comprise entre 0,01 et 5 % et de préférence entre 0,5 et 2 % en poids par rapport au poids total du vernis.

11. Vernis à ongles selon l'une quelconque des revendications précédentes, caractérisé par le fait qu'il contient en outre au moins un tensioactif fluoré hydrosoluble.

12. Vernis à ongles selon l'une quelconque des revendications précédentes, caractérisé par le fait qu'il contient en outre au moins un agent mouillant, un agent dispersant, un anti-mousse, un filtre solaire, un conservateur, un accélérateur de séchage, une cire, une silicone ou un mélange ceux-ci.

13. Procédé de maquillage des ongles, caractérisé par le fait qu'il consiste à appliquer sur la surface des ongles, un vernis selon l'une quelconque des revendications 1 à 12.

14. Utilisation pour la préparation d'un vernis à ongles aqueux, coloré ou incolore, de particules de polyester-polyuréthanne anionique à l'état dispersé, dont la taille des particules est comprise entre 2 et 40 nm, et la dureté, mesurée au pendule de Persoz, du film obtenu après séchage, durant 24 heures à 30°C et à 50% d'humidité relative, d'une couche de 300 µm d'épaisseur d'une dispersion aqueuse à 28% de matière sèche desdites particules, est comprise entre 50 et 300 secondes.

## Claims

1. Coloured or colourless aqueous nail varnish, characterized in that it contains dispersed anionic polyester-polyurethane particles with a particle size of between 2 and 40 nm, and for which the hardness, measured using a Persoz pendulum, of the film obtained after drying, for 24 hours at 30°C and 50% relative humidity, of a layer 300 µm in thickness of an aqueous 28% dispersion of solids of the said particles is between 50 and 300 seconds.

2. Aqueous nail varnish according to Claim 1, characterized in that the size of the said particles is between 2 and 30 nm.

3. Aqueous nail varnish according to either of the preceding claims, characterized in that the hardness as defined in Claim 1 is between 80 and 250 seconds.

4. Nail varnish according to any one of the preceding claims, characterized in that the proportion of dispersed polyester-polyurethane particles in the said nail varnish is between 3 and 50%, and preferably between 10 and 50%, by weight relative to the total weight of the varnish.

5. Nail varnish according to any one of the preceding claims, characterized in that it also contains dispersed anionic polyether-polyurethane particles, the size of the said particles being between 30 and 500 nm and preferably between 50 and 150 nm.

6. Nail varnish according to Claim 5, characterized in that the weight proportion of the said polyether-polyurethane particles is less than that of the polyester-polyurethane particles.

7. Nail varnish according to any one of the preceding claims, characterized in that it also contains at least one thickener in a proportion of between 0.01 and 5%, and preferably between 0.01 and 1%, by weight relative to the total weight of the varnish.

8. Nail varnish according to Claim 7, characterized in that the said thickener is chosen from the group consisting of cellulose and its derivatives, silicates, clays, synthetic polymers and natural gums.

9. Nail varnish according to Claim 8, characterized in that the said thickener is chosen from the group consisting of hydroxyethylcellulose, laponite and associative polyurethanes.

10. Nail varnish according to any one of the preceding claims, characterized in that it also contains at least one organic or inorganic pigment in a proportion of between 0.01 and 5%, and preferably between 0.5 and 2%, by weight relative to the total weight of the varnish.

11. Nail varnish according to any one of the preceding claims, characterized in that it also contains at least one water-soluble fluoro surfactant.

12. Nail varnish according to any one of the preceding claims, characterized in that it also contains at least one wetting agent, a dispersing agent, an anti-foaming agent, a sunscreen, a preserving agent, a drying accelerator, a wax, a silicone or a mixture thereof.

13. Process for making up the nails, characterized in that it consists in applying a varnish according to any one of Claims 1 to 12 to the surface of the nails.

14. Use, for the preparation of a coloured or colourless aqueous nail varnish, of dispersed anionic polyester-polyurethane particles, with a particle size of between 2 and 40 nm, and for which the hardness, measured using a Persoz pendulum, of the film obtained after drying, for 24 hours at 30°C and 50% relative humidity, of a layer 300 µm in thickness of an aqueous 28% dispersion of solids of the said particles is between 50 and 300 seconds.

## Patentansprüche

1. Wäßriger, gegenbenenfalls gefärbter Nagellack, dadurch gekennzeichnet, daß er Teilchen aus anionischem Polyesterpolyurethan in dispergiertem Zustand enthält, wobei die Teilchengröße zwischen 2 und 40 nm liegt und die mit der Uhr nach Persoz gemessene Härte des nach dem Trocknen während 24 Stunden bei 30°C und 50 % relativer Feuchte erhaltenen Filmes einer Schichtdicke von 300 µm aus einer wäßrigen Dispersion mit 28 % Trockensubstanzgehalt an diesen Teilchen zwischen 50 und 300 Sekunden beträgt.

2. Wäßriger Nagellack nach Anspruch 1, dadurch gekennzeichnet, daß die Größe dieser Teilchen im Bereich zwischen 2 und 30 nm liegt.

3. Wäßriger Nagellack nach einem der vorstehenden Ansprüche, dadurch gekennzeichnet, daß die Härte gemaß Definition in Anspruch 1 zwischen 80 und 250 Sekunden liegt.

4. Nagellack nach einem der vorstehenden Ansprüche, dadurch gekennzeichnet, daß das Mengenverhältnis der Teilchen aus Polyesterpolyurethan in dispergiertem Zustand in diesem Nagellack im Bereich zwischen 3 und 50 Gew.-%, vorzugsweise zwischen 10 und 50 Gew.-% in Bezug auf das Gesamtgewicht des Lackes beträgt.

5. Nagellack nach einem der vorstehenden Ansprüche, dadurch gekennzeichnet, daß er zusätzlich noch Teilchen aus anionischem Polyetherpolyurethan in dispergiertem Zustand enthält, wobei die Größe diese Teilchen zwischen 30 und 500 nm, vorzugsweise zwischen 50 und 150 nm liegt.

6. Nagellack nach Anspruch 5, dadurch gekennzeichnet, daß der Gewichtsanteil dieser Polyetherpolyurethanteilchen geringer ist als der Anteil an Polyesterpolyurethanteilchen.

7. Nagellack nach einem der vorstehenden Ansprüche, dadurch gekennzeichnet, daß er zusätzlich noch mindestens ein Verdickungsmittel in einem Mengenverhältnis enthält, das im Bereich zwischen 0,01 und 5 Gew.-%, vorzugsweise zwischen 0,01 und 1 Gew.-% in Bezug auf das Gesamtgewicht des Lackes beträgt.

8. Nagellack nach Anspruch 7, dadurch gekennzeichnet, daß dieses Verdickungsmittel aus der aus Zellulose und deren Derivaten, den Silicaten, Tonsorten, synthethischen Polymeren und natürlichen Gummisorten bestehenden Gruppe ausgewählt ist.

9. Nagellack nach Anspruch 8, dadurch gekennzeichnet, daß das Verdickungsmittel aus der aus Hvdroxyethylzellulose, Laponit und kombinierten Polyurethanen bestehenden Gruppe ausgewählt ist.

10. Nagellack nach einem der vorstehenden Ansprüche, dadurch gekennzeichnet, daß er zusätzlich noch mindestens ein organisches oder anorganisches Pigment in einem Mengenverhältnis enthält, das im Bereich zwischen 0,01 und 5 Gew.-%, vorzugsweise zwischen 0.5 und 2 Gew.-% in Bezug auf das Gesamtgewicht des Lackes beträgt.

11. Nagellack nach einem der vorstehenden Ansprüche, dadurch gekennzeichnet, daß er zusätzlich noch mindestens ein wasserlösliches, fluoriertes Tensid enthält.

12. Nagellack nach einem der vorstehenden Ansprüche, dadurch gekennzeichnet, daß er zusätzlich noch mindestens ein Benetzungsmittel, ein Dispergiermittel, einen Schaumbremser, eine Sonnenfiltersubstanz, ein Konservierungsmittel, einen Trocknungsbeschleuniger, ein Wachs, ein Silicon oder eine Mischung daraus enthält.

13. Verfahren zum Schminken der Nägel, dadurch gekennzeichnet, daß es aus der Applikation eines Lackes nach einem der Ansprüche 1 bis 12 auf der Nageloberfläche besteht.

14. Verwendung von Teilchen aus anionischem Polyesterpolyurethan in dispergiertem Zustand zur Herstellung eines wäßrigen, gegebenenfalls gefärbten Nagellackes, wobei die Teilchengröße im Bereich zwischen 2 und 40 nm liegt und die mit der Uhr nach Persoz gemessene Härte des nach dem Trocknen während 24 Stunden bei 30 °C und 50 %-iger relativer Feuchte enthaltenen Filmes einer Schichtdicke von 300 µm aus einer wässrigen Dispersion von 28 % Trockensubstanzgehalt an diesen Teilchen im Bereich zwischen 50 und 300 Sekunden liegt.
